# EUROPEAN PATENT SPECIFICATION

(11) **EP 1 883 436 B1**
(45) Date of publication and mention of the grant of the patent: **26.08.2009**
(21) Application number: 06744928.0
(22) Date of filing: 12.05.2006
(51) Int. Cl.: A61M 5/42, A61B 8/08, A61M 25/06

(54) **Cannula inserting system**
Kanüleneinführungssystem
Système d'insertion de canule

(30) Priority: 18.05.2005 EP 05300386
(43) Date of publication of application: 06.02.2008
(73) Proprietor: Koninklijke Philips Electronics N.V., 5621 BA Eindhoven (NL)
(72) Inventor: SCHWACH, Carole, c/o Société Civile SPID, F-75008 Paris (FR); NEERKEN, Sieglinde, c/o Société Civile SPID, F-75008 Paris (FR); LUCASSEN, Gerhardus, c/o Société Civile SPID, F-75008 Paris (FR); GEERLIGS, Marion, c/o Société Civile SPID, NL-75008 Paris (NL); DE VREEDE, Frederikus, c/o Société Civile SPID, F-75008 Paris (FR); HEKKENBERG, Robertus, c/o Société Civile SPID, F-75008 Paris (FR); EIKELENBERG, Nicole, c/o Société Civile SPID, F-75008 Paris (FR)
(74) Representative: Schouten, Marcus Maria
(86) International application number: PCT/IB2006/051500
(87) International publication number: WO 2006/123282

(56) References cited:
- WO-A-20/04082749
- WO-A-20/06064433
- GB-A- 2 400 176
- US-A- 3 998 210
- US-A- 4 029 084
- US-A1- 2003 060 716
- US-A1- 2003 233 046
- US-A1- 2004 236 224
- US-A1- 2005 027 185
- US-B1- 6 443 928

## Description

The present invention relates to the field of cannulation, hence to the insertion of a cannula or needle into the vascular system of a person or an animal.

Insertion of a cannula or a needle into a person's vascular system is an everyday task for physicians and has to be performed with high accuracy and care. Therefore, a medical personnel has to be highly skilled for tasks like blood withdrawal, drug delivery or infusions. The physician has to fmd an appropriate blood vessel and to introduce a distal end of a cannula or a needle with a very high precision in order to prevent generation of hematoma or effusions. Depending on the vascular system of a person even a highly skilled and experienced physician may require several attempts to insert a needle or a cannula at a suitable location into a blood vessel. Such multiple attempts of puncturing are rather painful and cause an appreciable discomfort for the patient. Moreover, such multiple attempts are also rather time intensive, which is disadvantageous especially in emergency situations.

There exist various devices and systems providing needle or cannula guiding and that assist a physician for inserting the cannula or needle in the vascular system of a patient.

US 2003/0060716 A1 discloses a cannula insertion system of a pistol-like shape. The system comprises means for locating a blood vessel below the skin surface. The system is moved manually over the skin until a display indicates that an optimal position for puncturing has been found.

US 2005/0020918 A1 discloses an ultrasound device comprising a flexible ultrasonic sensor web covering a portion of a patient's body. The sensor web serves to acquire an image of structures within the body. It may contain apertures to insert elongate diagnostic or therapeutic instruments such as laparoscopic surgical instruments to perform a surgical operation.

US 2005/0027185 discloses a method for locating a blood vessel, the method includes transmitting waves into a body part through which a blood vessel runs, detecting reflections of the waves, determining a location of the blood vessel responsive to detecting the reflections of the waves, and providing a visual indication at a location that is adjacent to the blood vessel.

It is an object of the invention to provide a cannula insertion system which facilitates the cannulation of a preselected blood vessel.

The present invention provides a puncture system for inserting a needle or cannula into a blood vessel of a person or an animal as defined in the independent claim. The puncture system comprises location determination means providing at least one location of the blood vessel. The location identification means is mechanically flexible such that its shape is adaptable to the shape of the body of the patient or animal. The system further comprises processing means for determining a puncture location of the blood vessel, hence an optimal location of the blood vessel that is suitable for a needle or cannula insertion. This puncture location is determined by making use of output signals of the location determination means.

The flexible adaptation of the location determination means provides a major advantage with which the above-mentioned object can be achieved. This advantage consists in its shape which is adaptable to the shape of the body part of the patient or animal. In operation, the location determination means is put onto the skin and consequently, the location determination means has got a fixed spatial relationship with respect to the skin. Indeed, once the preselected blood vessel has been identified by the location determination means this blood vessel is always within its measurement range. This means that, if the localization determination means comprises imaging system, that the imaging system provides a steady picture of the blood vessel and its surrounding tissue. This contrast with rigid location determination means of the prior art which is difficult to hold in position, in particular when curved and/or small body parts such as the arm, the foot or the hand should be accessed for needle insertion. Furthermore, more body sites can be used for needle or cannula insertion as the above-mentioned body parts become accessible as well.

Another advantage of the flexible adaptation is that finding the appropriate blood vessel becomes easier. The location determination means can now be moved easily and in a smooth fashion over the skin when searching for the preselected blood vessel. Abrupt wobbling thus becomes less likely.

In an embodiment the location determination means has got at least one aperture for the insertion of a cannula into the body of the patient or animal. This allows using location determination means having a larger surface, for example by wearing it like a T-shirt in a similar fashion as described in the above-mentioned US 2005/0020918 A1. With a smaller size the measurement range is smaller and the cannula will have to be inserted from the boundary.

Alternatively, there may be no aperture in the location determination means. The puncture location can be separated from the localization determination means. In this case the puncture location is close to the localization means, e.g. at its border. The needle or cannula is then inserted under a certain angle with respect to the skin surface in order to reach the proper location within the tissue. This configuration is advantageous as the needle or cannula does not penetrate the localization means, especially when the flexible localization means is a single array or element.

In another embodiment there is a display on top of the location determination means, the display being adapted to visualize the blood vessel's course within the body of the person or animal. For that purpose the flexible display disclosed in WO 0427746 can be chosen. Such a display makes it easier to ensure that a located blood vessel stays within the measurement range of the location determination means when it is rigidly attached to the body or the patient or animal.

Typically, the location determination means is adapted to provide a plurality of geometric data of the blood vessel. This allows to determine parameters, such like blood vessel diameter, blood vessel size as well as a depth under the skin. Further, the location determination means effectively provides determination of the blood vessel's course. When an effective use of such geometric and location information of the blood vessel is made this allows the determination of an optimal puncture location with a high accuracy and reliability that finally allows to minimize a danger of injury of a vessel wall. Consequently generation or severity of bleeding, hematoma or inflammation can be reduced to a minimum. Also by effective usage of obtained geometric and location data of the blood vessel, multiple attempts for a needle or cannula insertion can be prevented, because the reliable and accurate inspection of the blood vessel prior to insertion of the needle or cannula nearly guarantees that the needle or cannula can be correctly inserted or introduced into the vascular system with a single attempt. In particular, in emergency situations it is obvious, that this guided puncture is highly advantageous compared to an entirely manual cannulation.

According to a further embodiment, the location determination means is not only adapted to detect and to identify a blood vessel underneath the skin surface but also provides location determination of the cannula's distal end. Hence, by means of the location determination means, it can be precisely checked whether the cannula or needle has been correctly inserted into the vascular system of the person or animal. Additionally, the puncture system comprises indication means that is adapted to indicate whether the cannula or needle has been correctly inserted into the blood vessel by the puncture system.
According to an embodiment the system comprises fixing means. The fixing means is associated with the location determination means and allows to secure a fixed position of the location determination means with respect to the body of the patient or animal. In this way there is no need that the operator uses its hand to fix the position of the location determination means which facilitates a hand-free operation.

The puncture system described above allows the manual insertion of a cannula and helps to guarantee a safe, comfortable and fast cannulation. However, the puncture system can be complemented by additional modules providing a highly automated needle and cannula insertion which is reliable, less painful, and does not require a skilled handling of the user.

For that purpose the puncture system has fastening means for fixing the needle or cannula with respect to the puncture system. The fastening means is moveable with respect to an inserting direction and at least with respect to a second direction that is substantially non-parallel to the inserting direction.

The inserting direction is typically given by the alignment of the needle or cannula and determines an angle at which the needle or cannula is intended to emerge the vascular system. Further, the fastening means is also moveable along at least a second direction, such as e.g. a direction being substantially parallel to the surface of the skin of the person or animal. Hence, the needle or cannula is moveable with respect to the location determination means as well as with respect to a blood vessel.

Furthermore, the fastening means provides manual movement and alignment of the needle or cannula and provides manual insertion of the distal end of the needle or cannula into the blood vessel at the puncture location. In essence, this provides semi automated insertion of the needle or cannula. The puncture system autonomously allocates and determines a suitable puncture location and guides an operator to insert the needle or cannula at the specified location into the blood vessel. The actual insertion is operator supported. The force required for inserting the needle or cannula into the blood vessel is then provided by the operator, thus guaranteeing a maximum of sensitivity during needle or cannula insertion.

For instance, the location determination means and the processing means serve to locate and to identify a blood vessel and to determine the puncture location, but insertion of the needle or cannula is not performed in a completely automated but in a way that is at least partially controlled by the operator. For example, the puncture system may direct the needle or cannula along the inserting direction and may also translate the cannula to the inserting position whereas insertion of the cannula or needle, i.e. translation of the cannula or needle along the inserting direction is performed operator supported.

According to a further embodiment of the invention, the location means is further adapted to track the location of the needle's or cannula's distal end during insertion of the needle or cannula. The puncture system has got control means for controlling the movement of the needle or cannula in response to the tracking of the needle's or cannula's distal end. In this way, the puncture system is provided with a feedback allowing to monitor and to check whether the distal end of the needle or cannula is correctly inserted. This functionality effectively represents a safety mechanism of the puncture system and helps to prevent that despite of an accurate inspection of the blood vessel the cannula might be incorrectly introduced, which may have serious consequences for the person's or animal's health.

Typically, the location determination means provides course and location determination of the blood vessel as well as tracking of the needle's or cannula's distal end at a sufficient repetition rate that allows for fast reaction in case that the cannula introduction deviates from a determined path or schedule. Also, the location determination means allows to check whether the distal end of the needle or cannula has been inserted correctly into the person's vascular system. Hence, the location determination means not only provides a control mechanism during needle or cannula insertion but also allows to check the final position of the needle or cannula after the intra vascular inserting has been terminated.

Instead of tracking the needle's or cannula's distal end it is also possible to monitor and follow the position or movement of the blood vessel during insertion. In principle this should also provide enough information and is a somewhat simpler solution compared to cannula tracking. If it is known where the needle or cannula has to end up and if the insertion parameters have been determined, the location of the blood vessel can be monitored during insertion. The insertion however goes wrong if the blood vessel does not stay in place.

According to an embodiment, the puncture system further comprises actuation means that is adapted to autonomously move the fastening means into an inserting position and that is further adapted to insert the distal end of the needle or cannula into the blood vessel at the puncture location. In this embodiment the invention provides an entirely automated needle or cannula insertion. Hence, the inventive puncture system provides a location determination of the blood vessel, an autonomous alignment and movement of the cannula to an inserting position and inserting direction and finally an automated inserting of the cannula into the blood vessel at a location determined by the puncture system itself.

In this sense the inventive puncture system provides an entirely automated insertion of a needle or cannula into a person's or animal's blood vessel, which is applicable to e.g. blood sampling or blood withdrawal, drug medication or infusion, blood transfusion, general catheter insertion and dialysis. The entire process of locating of a blood vessel, determining of a puncture location as well as mechanically shifting and aligning the needle or cannula and finally inserting of the needle or cannula can be performed without any user interaction, allowing to execute the above mentioned tasks in an highly automated manner that may even be performed by unskilled or low-skilled medical personnel.

According to a further embodiment, the actuation means is manually controllable by an operator for manual insertion of the cannula by the operator. Even though if implemented as an autonomous system, the puncture system also allows for a partially automated insertion of a needle or cannula.

According to a further preferred embodiment of the invention, the location means comprises optic and/or acoustic or opto-acoustic detection or signaling means and corresponding processing means that is adapted to perform a corresponding signal acquisition and signal processing for deriving relevant parameters for determination of the puncture location. The detection or signaling means may be realized as imaging and image processing means but may also be implemented as an acquisition system making use of e.g. Doppler based techniques that do not require image acquisition and respective image processing.

Typically, the location determination means is realized by making use of techniques, such as Near infrared imaging, Optical Coherence Tomography, Photo Acoustic Imaging or Ultrasound Techniques. In particular, Ultrasound techniques, Optical Coherence Tomography and Photo Acoustic Imaging provide inspection and analysis of the blood vessel and allow for a precise acquisition of blood vessel related data, even if the blood vessel is located at an appreciable depth under the skin of the person. Other techniques, that might be based on Doppler signals, like Doppler Ultrasound or Doppler Optical Coherence Tomography that are adapted to locate a flowing or streaming liquid, such as blood flowing in a blood vessel, in principle also allow a precise and reliable location determination of a blood vessel underneath the skin surface. Also, combinations of Doppler- based and imaging based signal acquisition techniques might be implemented.

In a further embodiment, the processing means is further adapted to determine the inserting position in response to the puncture position, which in turn is obtained by means of an optimization procedure performed by the processing means, that accounts for at least one blood vessel parameter that is derived from a data acquisition performed by the location determination means. The optimization procedure is preferably performed by making use of a plurality of blood vessel parameters, such as blood vessel size, blood vessel diameter, location and depth underneath the skin surface, the course of the blood vessel as well as the general geometry of the blood vessel. For example, the processing means is supplied with graphical data processing means in order to recognize and identify particular regions of a blood vessel that are not suitable for puncture, such as regions of a blood vessel that are near a branch or a junction. Also, the processing means and the location determination means may determine regions of a blood vessel featuring a narrowing that might be due to calcification. Hence, the optimization procedure serves to determine an optimal location of the blood vessel that is suitable for puncturing and that is expected to cause a minimum of discomfort or pain.

The optimization procedure determines the optimal puncture position of the blood vessel but it may further be applied to determine a corresponding inserting position for the cannula that is governed by the puncture position as well as by the inserting direction of the cannula. The inserting direction, hence the angle at which the needle or cannula is to be inserted into the blood vessel, is also preferably determined by means of the optimization procedure and typically varies between 10^{°} and 45^{°}with respect to the person's skin. Depending on the blood vessel's depth underneath the skin surface and the inserting direction, the inserting position may substantially deviate from the puncture position of the blood vessel.

According to a further embodiment, the orientation of the needle or cannula and the inserting direction are modifiable and the actuation means are adapted to translate and to align the fastening means in three spatial directions. In this way, the needle or cannula may be freely arranged and oriented within a range of coverage of the puncture system and may therefore be arbitrarily positioned as determined by the processing means and the puncture location.

According to a further embodiment, the needle or cannula is applicable to blood withdrawal and/or drug infusion and/or blood transfusion, and/or catheter insertion and/or dialysis applications. Hence, the invention can be universally applied to various different medical purposes that require insertion of a needle or cannula into a vascular system of a person. Respective fastening means for fixing the needle or cannula are typically realized by making use of a modular concept allowing for a quick and secure adaptation of the needle or cannula inserting system to a multitude of different purposes.

In another embodiment, the invention provides autonomous insertion of the needle's or cannula's distal end into the blood vessel in response of detecting that the needle or cannula is at the insertion position. Hence, insertion of the needle or cannula is typically performed by means of a two step process. In a first step the needle or cannula is translated and moved as well as aligned in the inserting position and along the inserting direction and thereafter in the second step any movement of the needle or cannula is disabled except for a movement along the inserting direction. In this way, it can be effectively guaranteed, that during cannula insertion a potentially harmful lateral movement of the needle or cannula is prevented. Hence, during cannula insertion the lateral position of the cannula remains substantially fixed with respect to the surface of the skin, i.e. the needle or cannula is only moved along the inserting direction.

In a further embodiment, the system further comprises a movement control unit that is adapted to process data provided by the acquisition system during insertion of the cannula in order to control the needle's or cannula's movement during insertion into the tissue of the person or animal.

In a further embodiment, the puncture system is adapted to insert the needle or cannula and to be detached from the inserted needle or cannula in such a way, that the puncture system is removable from the body part leaving the needle or cannula inserted in the biological tissue, e.g. in the skin. Therefore, the fastening means are adapted to dissociate the needle or cannula, if the needle or cannula has been correctly inserted. Further, if the needle or cannula is correctly inserted the cuff can be loosened, thus allowing to detach the entire puncture system from the body part. This feature is particularly advantageous with respect to catheter as well as infusion related application scenarios.

In the following various embodiments of the invention will be described in more detail by making reference to the drawings in which:
- Figure 1: illustrates a schematic block diagram of the inventive puncture system,
- Figure 2: shows a schematic illustration of puncture location and inserting position determined by the puncture system,
- Figure 3: shows attachment of the puncture system to a suitable body part,
- Figure 4: shows a flowchart of determining puncture location and performing needle or cannula insertion,
- Figure 5: shows a flowchart of needle or cannula insertion with skin penetration detection.

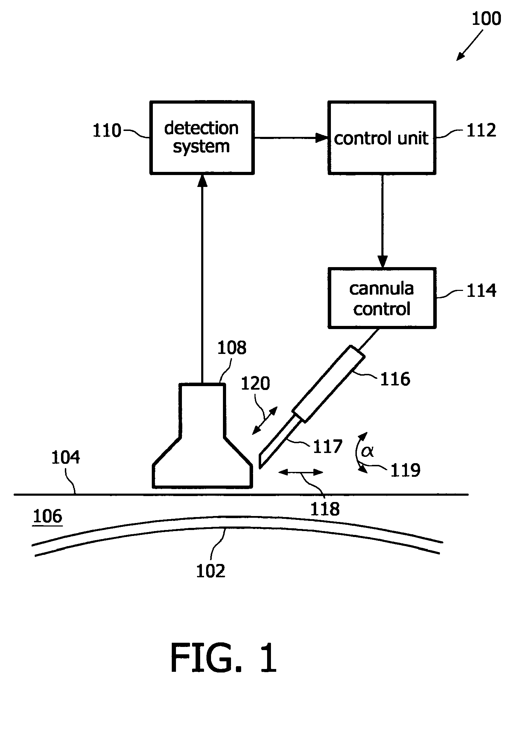

Fig. 1 shows a schematic block diagram of the puncture system 100. The puncture system 100 has an acquisition module 108, a detection system 110, a control unit 112, a cannula control 114 as well as an optional cannula mount 116.

The cannula 117 itself can be rigidly attached to the cannula mount 116 that represents fastening means for fixing the cannula and means for moving and aligning the cannula 117 as controlled by the cannula control unit 114. The cannula 117 or the cannula mount 116 can be moved along the inserting direction 120 as well as along direction 118 that is substantially parallel to the surface of the skin 104. In principle direction 118 can be any direction in the plane parallel to the skin surface. Typically, the cannula 117 and the cannula mount 116 are moveable by means of the cannula control 114 in all three spatial directions. Also, the angle α 119 between the inserting direction 120 and the surface of the skin 104 may be arbitrarily modified by means of the cannula control 114 in a way that is determined by means of the detection system 110 and the control unit 112. As mentioned above the cannula mount 116 represents the fastening means and are optional. Without the cannula mount the cannula 117 is inserted manually. The description which follows uses the fastening means for the provision of a semi-automatic or fully automatic puncture system.

Fig. 1 shows application of the puncture system to a person by means of a cross sectional illustration of the person's skin 104. Underneath the surface of the skin 104 there is located a blood vessel 102 that is surrounded by tissue 106. When the puncture system 100 is attached to the skin 104 of the person, the acquisition module 108 is adapted to acquire optical, opto-acoustic or acoustic data from the tissue 106 and the blood vessel 102 that allows to classify at least one blood vessel parameter, such as location of the blood vessel, diameter of the blood vessel, size of the blood vessel, depth underneath the surface of the skin 104, geometry of the blood vessel, blood flow or similar parameters.

Preferably, the acquisition module 108 is realized by means of Ultrasound, Near-infrared imaging, Optical Coherence Tomography, Doppler Ultrasound, Doppler Optical Coherence Tomography or Photo Acoustic techniques that allow to generate a signal providing identification of the blood vessel 102. Signals acquired by the acquisition module 108 are provided to the detection system 110, which in turn generates a signal of the blood vessel 102. Hence, detection system 110 as well as acquisition module 108 are coordinated in a sense that the detection system 110 is suitable to perform signal processing of signals obtained from the acquisition module 108. By making use of optical, opto-acoustic or ultrasound detection, the blood vessel 102 may be precisely located even at an appreciable depth underneath the surface of the skin 104. Additionally or alternatively also Doppler techniques may be applied including e.g. Doppler Ultrasound techniques allowing for detection of e.g. blood flow in the blood vessel 102. Also, Doppler Optical Coherence Tomography might be correspondingly applied.

Acquisition of location data, geometric data as well as data related to the course of the blood vessel 102, may also be obtained without an imaging of the blood vessel. Therefore, the imaging system 110 does not necessarily have to provide a visual image. Instead the imaging system 110 may be enabled to directly extract blood vessel parameters from the signals acquired by the acquisition module 108. Hence, extraction of blood vessel parameters may be performed by means of the detection system 110 or by the control unit 112

The control unit 112 has a processing unit that is enabled to process the data obtained from the detection system 110. Depending on the type of data provided by the detection system 110, the processing unit of the control unit 112 may further process blood vessel parameters in order to extract required blood vessel parameters from a signal of the blood vessel 102.

The control unit 112 serves to process the blood vessel parameters in order to find and to determine a puncture location of the blood vessel 102 that is ideally suited for an insertion of the cannula 117. In a basic embodiment this puncture location may be determined with respect to location and course of the blood vessel 102. More sophisticated implementations further account for the vessel geometry in the vicinity of an intended puncture location as well as vessel diameter and depth underneath the surface of the skin 104.

Typically, the puncture location may be determined as a result of an optimization procedure taking into account all kinds of blood vessel parameters. For instance, the optimization procedure that is typically performed by means of the processing unit of the control unit 112 may specify, that a puncture location must not be in the vicinity of a branch or junction of a blood vessel 102. Further, a puncture location may require a certain diameter of the blood vessel 102. Also, the puncture location may be determined with respect of a smallest possible depth of the blood vessel 102 underneath the surface of the skin 104. Additionally, the control unit may also determine the inserting direction 120 specifying at which angle α 119 the cannula 117 has to be introduced into the skin 104 and the tissue 106.

Having determined the puncture location, the control unit 112 is further adapted to specify an inserting position for the cannula 117. The inserting position specifies a position as well as an alignment or direction of the cannula 117 from which the cannula 117 has to be shifted along the inserting direction, i.e. the direction coinciding with the longitudinal direction of the cannula, in order to impinge into the blood vessel at the determined puncture location with its distal end.

After determination of puncture location and inserting position, the control unit 112 generates corresponding driving signals to the cannula control 114, which in turn is adapted to move the cannula mount 116 into a corresponding position. The cannula control 114 is typically rigidly connected to the cannula mount 116 and is provided with e.g. electro-mechanical means, such as electromotive actuators, like e.g. piezo driven actuators that allow for a precise electronically controlled mechanical movement of the cannula 117. Typically, the cannula control 114 provides lateral displacement of the cannula mount 116 as well as shifting the cannula mount 116 or the cannula 117 along the inserting direction 120.

As soon as the cannula 117 advances towards the blood vessel 102, the acquisition module 108 also acquires position data of the distal end of the cannula 117. Especially, when the cannula 117 already penetrated the skin 104, detection of its distal end allows to control the movement of the cannula 117 through the tissue. As soon as the acquisition module 108 detects, that the distal end of the cannula 117 does not properly hit the blood vessel 102, the entire process of cannula insertion may be aborted and the cannula 117 might be withdrawn. In this way, simultaneous acquisition of blood vessel related data and position data of the distal end of the cannula 117 allows to effectively realize a feedback and security mechanism for the autonomous puncture system.

Instead of tracking the needle's or cannula's distal end it is also possible to monitor and follow the position or movement of the blood vessel during insertion. In principle this should also provide enough information and is a somewhat simpler solution compared to cannula tracking. If it is known where the needle or cannula has to end up and if the insertion parameters have been determined, the location of the blood vessel can be monitored during insertion. The insertion however goes wrong if the blood vessel does not stay in place.

Since the puncture system 100 is principally applicable to any kind of puncture related applications, like blood sampling, blood withdrawal, infusion or medication, the cannula can be effectively replaced by a needle or catheter that are specific for dedicated applications, respectively.

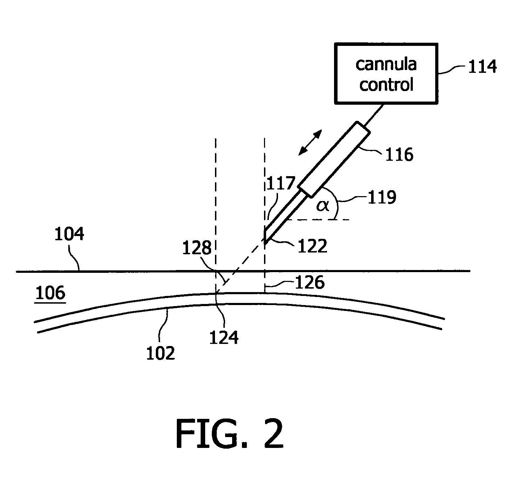

Fig. 2 shows a schematic illustration of puncture location 124 and the inserting position 126 that are determined by the puncture system. Similar to Fig. 1 also a cross sectional view of a person's or animal's skin is shown. The puncture system 100 determines the puncture location 124 of the blood vessel 102 by making use of blood vessel parameters that were obtained by means of the acquisition module 108 and corresponding detection and processing means. Here, the blood vessel 102 shows a uniform diameter and the puncture location 124 is determined by a position of the blood vessel 102 that is closest to the surface of the skin 104. This puncture location 124 may also be chosen by an experienced physician for manually inserting the cannula into the blood vessel 102. Hence, the inventive puncture system aims to determine a puncture location that provides a minimum of discomfort and pain as well as a minimum of danger of injury to the vessel wall, which may have severe consequences for the health status of the person.

Furthermore, the control unit 112 may also determine an optimal insertion angle α 119 that determines the insertion direction 120 of the cannula 117. Since the cannula 117 is typically introduced at a non-perpendicular angle with respect to the surface of the skin 104, the point of penetration through the skin 104 and the puncture location 124 typically describe an insertion path 128 for the cannula 117 that coincides with the inserting direction 120. Before advancing the cannula 117 into the skin 104, it has to be moved to the inserting position 126 featuring a lateral displacement from the puncture location 124. In this context, lateral displacement is to be interpreted as a displacement in the plane of the surface of the skin 104. For instance, the inserting position 126 may be determined as the position where the distal end of the cannula 122 coincides with the insertion path 128.

Insertion of the needle is preferably performed as a two step process, wherein the first step is given by moving the cannula 117 to the inserting position 126 by means of the cannula control unit 114. As soon as the inserting position 126 has been reached by the distal end of the cannula 122, the second step of advancing and inserting the cannula 117 into the skin 104, the tissue 106 and finally into the puncture location of the blood vessel 102 is initialized. Advancing and inserting of the cannula 117 is controlled by means of the acquisition module 108 and the control unit 112 in order to correct the movement of the cannula 117 during the insertion process. However, as soon as the acquisition module 108 detects, that the distal end of the cannula 122 has penetrated through the surface of the skin 104, a lateral movement of the cannula by means of the cannula control 114 is disabled for preventing severe injury of the skin 104 and the tissue 106.

Additionally, the puncture system 100 may be provided with a release module in order to manually control the insertion of the cannula 117. Then, a user of the puncture system may manually trigger the advancing of the cannula 117 along the inserting direction 120 and may manually control whether the inserting position 126 and/or the puncture location 124 determined by a puncture system are reasonable for inserting a cannula.

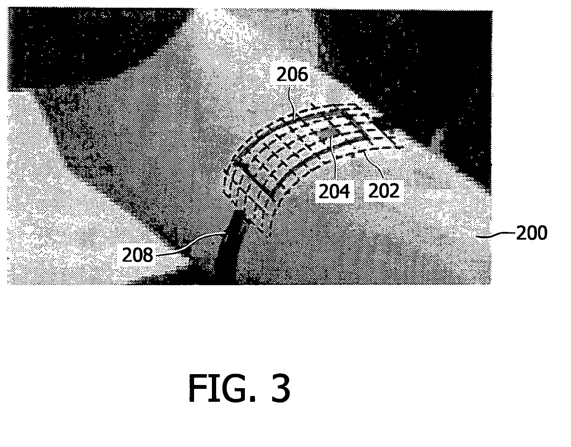

Fig. 3 schematically shows an embodiment of the puncture system applied to a body part of a person or an animal.
The location determination means 202 of the puncture system is fastened by a strap 208 to a body part (the arm) 200 of a patient. The location determination means 200 is flexible and follows the curvature of the body part 200. It has a display 206 on top and an aperture 204 for inserting a cannula. The processing unit (not shown) may be located within the flexible location determination means. In the alternative the data from the location determination means 202 are transferred to an external processing unit and are displayed on an external display (not shown).

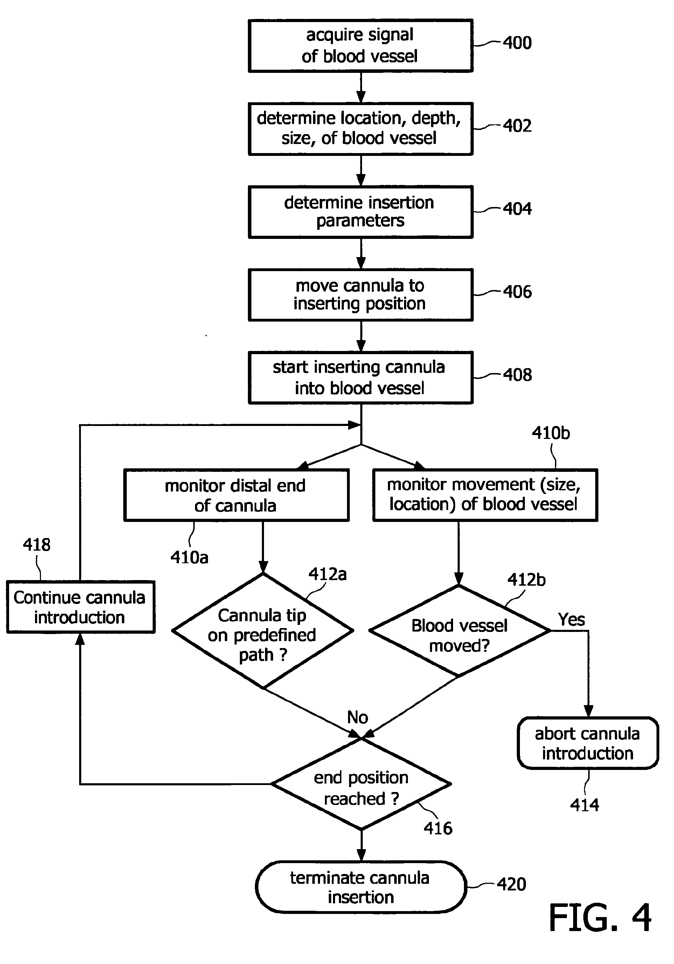

Fig. 4 is illustrative of a flowchart of determining a puncture and inserting position and for performing the needle or cannula insertion. In a first step 400 a signal of the blood vessel 102 is acquired by means of the acquisition module 108. The acquired data is then processed by means of the processing unit of the control unit 112 in order to determine e.g. location, depth, size, course and general geometric data of the blood vessel. Based on these blood vessel parameters representing blood vessel location and general properties of the blood vessel, in step 404 insertion parameters for the needle or cannula insertion are determined, preferably by means of an optimization procedure performed by the control unit 112. The insertion parameters represent at least the puncture location 124 of the blood vessel 102. Preferably, the insertion parameters also specify the inserting position 126 as well as the inserting direction given by the angle 119.

After the insertion parameters have been determined, the cannula is moved in step 406 to the insertion position. Hence, the needle or cannula is aligned along the inserting direction as well as laterally displaced to the inserting position 126. If the distal end 122 of the needle or cannula has reached the determined inserting position, in step 408 the insertion of the needle or cannula into the blood vessel starts. During this movement the following steps 410a or 410b through 420 are performed.

### To follow needle insertion at least two alternative possibilities can be chosen:

A first possibility is as follows:
During the advancing of the needle or cannula along the inserting direction 120, the distal end of the needle or cannula is monitored in step 410a by means of the acquisition module 108 and corresponding signal processing means. In the successive step 412a, the distal end of the needle or cannula or the needle or cannula tip's position is compared with the trajectory of the insertion path 128. In cases where the position of the distal end of the cannula 117 clearly deviates from the calculated insertion path 128, the method continues with step 414, where the needle or cannula introduction or insertion is aborted and the needle or cannula is withdrawn from the tissue in the opposite direction. Otherwise, if in step 412a the distal end of the cannula coincides with the insertion path 128 at least within a predefined margin, the method continues with step 416 where the position of the distal end of the needle or cannula is compared with the calculated end position inside the blood vessel 102.

### A second possibility is as follows:

During the advancing of the needle or cannula along the inserting direction 120, the location and size of the blood vessel 102 is monitored in step 410b by means of the acquisition module 108 and corresponding signal processing means. In the successive step 412b, the blood vessel location and size is compared with its original location and size just before starting the insertion. In cases where the position and size of the blood vessel clearly deviates from its original location and size, the method continues with step 414, where the needle or cannula introduction or insertion is aborted and the needle or cannula is withdrawn from the tissue in the opposite direction. Otherwise, if in step 412b the location and size of the blood vessel coincides with its original parameters at least within a predefined margin, the method continues with step 416 where the location and size of the blood vessel is compared with the original parameters of the blood vessel 102.

If the end position has not yet been reached, the method continues with step 418, where the needle or cannula introduction continues. Thereafter, the method returns to step 410a or 410b, where the position of the distal end of the needle or cannula 122 is repeatedly monitored. It is to be noted that the movement of the needle or cannula is performed as a continuous movement, i.e. it is not performed step wise as the single steps of the flowchart in Fig. 4 may suggest. The loop described by steps 410a or 410b through steps 418 continues as long as the end position of the cannula has not been reached. As soon as in step 416 the end position of the cannula has been detected, the procedure continues with step 420, where the cannula insertion is terminated.

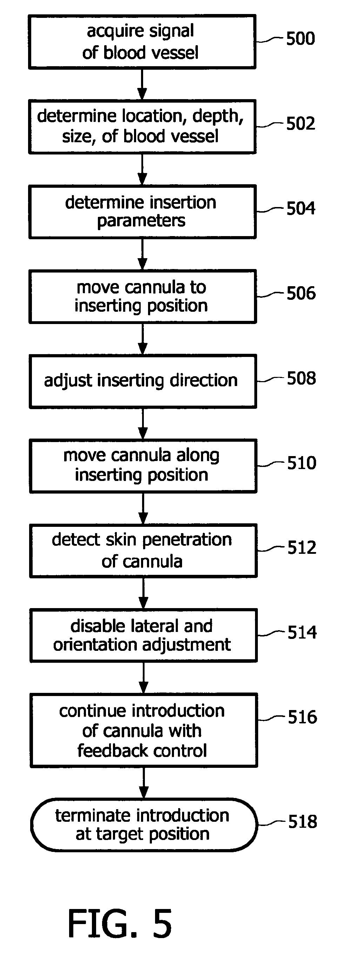

Fig. 5 is illustrative of a flowchart of introducing the needle or cannula into the blood vessel 102 making use of a skin penetration detection of the needle or cannula. Here, steps 500 through 504 correspond to steps 400 through 404 of Fig. 4. In step 506 the needle or cannula is moved to the inserting position by a translational movement controlled by the needle or cannula control unit 114. Thereafter, in step 508 the inserting direction of the cannula 117 is adjusted according to the determined inserting angle 119. Alternatively, needle or cannula insertion may also be performed by making use of a predefined insertion angle, thus allowing to reduce the complexity of the puncture system. In step 510 the movement of the cannula along the inserting direction is started, thereby making use of position determination of the cannula's distal end or controlling the inserting motion.

In step 512 the system detects the penetration of the skin of the person by the cannula and in the subsequent step 514 all lateral and orientational movement or adjustment of the cannula are disabled. Detection of skin penetration can be performed by means of the acquisition module 108 and its corresponding signal processing means. Also, the skin penetration might be detected by means of a pressure sensor being implemented in the cannula mount 116. Disabling of lateral cannula movement serves as an effective means for injury prevention. After lateral adjustment of the cannula has been disabled in step 514, in step 516 introduction or insertion of the cannula into the blood vessel is continued by making use of the feedback control mechanism illustrated by the flowchart of Fig. 4. As soon as the estimated end position of the cannula has been reached the procedure stops in step 518.

In general, cannula or needle insertion described by the flowchart of figure 5 can be performed with feedback mechanism providing the actual location of the cannula's distal end or checking possible displacement of the blood vessel, preferably during insertion of the cannula. However, the entire needle or cannula insertion process may also be performed without such a feedback mechanism, allowing to reduce the complexity of the puncture system. In this case, after determination of the puncture location as well as determination of required blood vessel parameters, needle or cannula insertion can be autonomously or manually performed without a continuous needle or cannula or blood vessel tracking just by making use of the determined blood vessel parameters.

## Claims

1. A puncture system (100) for inserting a cannula (117) into a blood vessel (102) of a person or animal comprising:
a) location determination means for determining at least one location of the blood vessel, the location determination means being mechanically flexible such that its shape is adaptable to the shape of a body part of the person or animal,
b) processing means for determining a puncture location (124) of the blood vessel depending on the output of the location determination means, the processing means being further arranged for determining an inserting position (126) for the cannula (117) specifying a position and alignment for the cannula (117).

2. The system according to claim 1, **characterized in that** the location determination means has got at least one aperture (204) for the insertion of a cannula into the body of the patient or animal.

3. The system according to claim 1, **characterized in that** there is a display on top of the location determination means, the display being adapted to visualize the blood vessel's course within the body of the person or animal.

4. The system according to claim 1, **characterized in that** the location determination means is further adapted to determine the location of the cannula's distal end (122).

5. The system according to claim 1, **characterized in that** it comprises first fixing means, the first fixing means being associated with the location determination means, the first fixing means securing a fixed position of the location determination means with respect to the body of the patient or animal.

6. The system according to claim 1, **characterized in that** it further comprises fastening means (116) for fixing the cannula, said fastening means being moveable along an inserting direction (120) and at least a second direction (118) being substantially non-parallel to the inserting direction, for insertion of the distal end (122) of the cannula into the blood vessel at the puncture location.

7. The system according to claim 6, **characterized in that** the location determination means is further adapted to track the location of the cannula's distal end (122) during insertion of the cannula (117) and that the system further comprises control means for controlling the movement of the cannula by means of fastening means (116) in response to the tracking of the cannula's distal end.

8. The system according to claim 6, **characterized in that** the location determination means is further adapted to track the at least one blood vessel parameter during insertion of the cannula (117) and that the system further comprises control means for controlling the movement of the cannula by means of fastening means (116) in response to the tracking of the blood vessel parameter.

9. The system according to claim 6, **characterized in that** it comprises actuation means being adapted to autonomously move the fastening means into an inserting position (126) and to insert the distal end (122) of the cannula into the blood vessel at the puncture location.

10. The system according to claim 1, **characterized in that** the location determination means comprise optic and/or acoustic or opto-acoustic detection and signal acquisition means (108, 110) and wherein the processing means (110, 112) is adapted to perform signal processing.

11. The system according to claim 10, **characterized in that** the detection and signal acquisition means (108, 110) are implemented by making use of image acquisition and image processing techniques and /or by making use of Doppler implemented location determination techniques.

12. The system according to claim 10, **characterized in that** the processing means is further adapted to determine the inserting position (126) in response to the puncture position (124) being obtained by means of an optimization procedure accounting for at least one blood vessel parameter being derived from the output of the location determination means.

13. The system according to claim 9, **characterized in that** the orientation of the cannula (117) and the inserting direction (120) are modifiable and that the actuation means are adapted to translate and align the fastening means in three spatial directions.

14. The system according to claim 1, **characterized in that** the insertion of the cannula's distal end (122) into the blood vessel (102) is autonomously performed in response of detecting that the cannula (117) is at the insertion position (126).

## Patentansprüche

1. Einstichsystem (100) zum Einführen einer Kanüle (117) in ein Blutgefäß (102) einer Person oder eines Tieres, wobei das Einstichsystem Folgendes umfasst:
a) Positionsbestimmungsmittel zum Bestimmen mindestens eines Ortes des Blutgefäßes, wobei das Positionsbestimmungsmittel mechanisch biegsam ist, so dass sich seine Form an die Form eines Körperteils der Person oder des Tieres anpassen kann,
b) Verarbeitungsmittel zum Bestimmen einer Einstichstelle (124) des Blutgefäßes abhängig von der Ausgabe des Positionsbestimmungsmittels, wobei das Verarbeitungsmittel weiterhin dafür vorgesehen ist, eine Einführungsposition (126) für die Kanüle (117) zu bestimmen und dabei eine Position und Ausrichtung für die Kanüle (117) zu spezifizieren.

2. System nach Anspruch 1, **dadurch gekennzeichnet, dass** das Positionsbestimmungsmittel mindestens eine Apertur (204) zum Einführen einer Kanüle in den Körper des Patienten oder des Tieres aufweist.

3. System nach Anspruch 1, **dadurch gekennzeichnet, dass** sich eine Anzeige über dem Positionsbestimmungsmittel befindet, wobei die Anzeige vorgesehen ist, um den Verlauf des Blutgefäßes im Körper der Person oder des Tieres zu visualisieren.

4. System nach Anspruch 1, **dadurch gekennzeichnet, dass** das Positionsbestimmungsmittel weiterhin dafür vorgesehen ist, die Position des distalen Endes (122) der Kanüle zu bestimmen.

5. System nach Anspruch 1, **dadurch gekennzeichnet, dass** es erste Fixierungsmittel umfasst, wobei die ersten Fixierungsmittel zu dem Positionsbestimmungsmittel gehören und eine feste Position des Positionsbestimmungsmittels in Bezug auf den Körper des Patienten oder Tieres sichern.

6. System nach Anspruch 1, **dadurch gekennzeichnet, dass** es weiterhin Befestigungsmittel (116) zum Fixieren der Kanüle umfasst, wobei die genannten Befestigungsmittel entlang einer Einführungsrichtung (120) und mindestens einer zweiten Richtung (118), die im Wesentlichen nicht parallel zu der Einführungsrichtung verläuft, bewegbar sind, um das distale Ende (122) der Kanüle bei der Einstichstelle in das Blutgefäß einzuführen.

7. System nach Anspruch 6, **dadurch gekennzeichnet, dass** das Positionsbestimmungsmittel weiterhin dafür vorgesehen ist, die Position des distalen Kanülenendes (122) beim Einführen der Kanüle (117) zu verfolgen, und dass das System weiterhin Steuermittel zum Steuern der Kanülenbewegung mit Hilfe von Befestigungsmitteln (116) in Reaktion auf das Verfolgen des distalen Kanülenendes umfasst.

8. System nach Anspruch 6, **dadurch gekennzeichnet, dass** das Positionsbestimmungsmittel weiterhin dafür vorgesehen ist, den mindestens einen Blutgefäßparameter während des Einführens des Kanüle (117) zu verfolgen, und dass das System weiterhin Steuermittel zum Steuern der Kanülenbewegung mit Hilfe von Befestigungsmitteln (116) in Reaktion auf das Verfolgen des Blutgefäßparameters umfasst.

9. System nach Anspruch 6, **dadurch gekennzeichnet, dass** es Betätigungsmittel umfasst, die vorgesehen sind, um die Befestigungsmittel autonom in eine Einführungsposition (126) zu bewegen und das distale Ende (122) der Kanüle bei der Einstichstelle in das Blutgefäß einzuführen.

10. System nach Anspruch 1, **dadurch gekennzeichnet, dass** die Positionsbestimmungsmittel optische und/oder akustische oder opto-akustische Detektions- und Signalerfassungsmittel (108, 110) umfassen und wobei das Verarbeitungsmittel (110, 112) vorgesehen ist, um eine Signalverarbeitung durchzuführen.

11. System nach Anspruch 10, **dadurch gekennzeichnet, dass** die Detektions- und Signalerfassungsmittel (108, 110) mit Hilfe von Bilderfassungs- und Bildverarbeitungstechniken und/oder mit Hilfe von Doppler-basierenden Ortsbestimmungstechniken implementiert werden.

12. Verfahren nach Anspruch 10, **dadurch gekennzeichnet, dass** das Verarbeitungsmittel weiterhin dafür vorgesehen ist, die Einführungsposition (126) in Reaktion auf die Einstichstelle (124) zu bestimmen, die mit Hilfe einer Optimierungsprozedur erlangt wurde, welche mindestens einen Blutgefäßparameter berücksichtigt, der aus der Ausgabe des Positionsbestimmungsmittels abgeleitet wird.

13. System nach Anspruch 9, **dadurch gekennzeichnet, dass** die Orientierung der Kanüle (117) und die Einführungsrichtung (120) modifizierbar sind und dass die Betätigungsmittel vorgesehen sind, um die Befestigungsmittel in drei räumlichen Richtungen zu verschieben und auszurichten.

14. System nach Anspruch 1, **dadurch gekennzeichnet, dass** die Einführung des distalen Kanülenendes (122) in das Blutgefäß (122) autonom in Reaktion darauf durchgeführt wird, dass erkannt wird, dass sich die Kanüle (177) an der Einführungsposition (126) befindet.

## Revendications

1. Système de ponction (100) pour insérer une canule (117) dans un vaisseau sanguin (102) d'une personne ou d'un animal comprenant :
a) des moyens de détermination de l'emplacement permettant de déterminer au moins un emplacement du vaisseau sanguin, les moyens de détermination de l'emplacement étant mécaniquement flexibles, de sorte que sa forme soit adaptable à la forme d'une partie du corps de la personne ou de l'animal,
b) des moyens de traitement pour déterminer un emplacement de ponction (124) du vaisseau sanguin, en fonction du rendement des moyens de détermination de l'emplacement, les moyens de traitement étant en outre disposés de façon à déterminer une position d'insertion (126) pour la canule (117), en spécifiant une position et un alignement pour la canule (117).

2. Système selon la revendication 1, **caractérisé en ce que** les moyens de détermination de l'emplacement ont au moins une ouverture (204) pour l'insertion d'une canule dans le corps du patient ou de l'animal.

3. Système selon la revendication 1, **caractérisé en ce qu'**il présente un écran sur la partie supérieure des moyens de détermination de l'emplacement, l'écran étant adapté pour visualiser le cours du vaisseau sanguin dans le corps de la personne ou de l'animal.

4. Système selon la revendication 1, **caractérisé en ce que** les moyens de détermination de l'emplacement sont en outre adaptés pour déterminer l'emplacement de l'extrémité distale de la canule (122).

5. Système selon la revendication 1, **caractérisé en ce qu'**il comprend des premiers moyens de fixation, lesdits premiers moyens de fixation étant associés aux moyens de détermination de l'emplacement, lesdits premiers moyens de fixation assurant une position fixe des moyens de détermination de l'emplacement par rapport au corps du patient ou de l'animal.

6. Système selon la revendication 1, **caractérisé en ce qu'**il comprend en outre des moyens d'attache (116) pour fixer la canule, lesdits moyens d'attache étant mobiles le long d'une direction d'insertion (120) et au moins une seconde direction (118) étant sensiblement non-parallèle à la direction d'insertion, pour l'insertion de l'extrémité distale (122) de la canule dans le vaisseau sanguin à l'emplacement de la ponction.

7. Système selon la revendication 6, **caractérisé en ce que** les moyens de détermination de l'emplacement sont en outre adaptés pour suivre l'emplacement de l'extrémité distale de la canule (122) pendant l'insertion de ladite canule (117) et **en ce que** le système comprend en outre des moyens de contrôle permettant de contrôler le mouvement de la canule à l'aide de moyens d'attache (116), en réponse au suivi de l'extrémité distale de la canule.

8. Système selon la revendication 6, **caractérisé en ce que** les moyens de détermination de l'emplacement sont en outre adaptés pour suivre le au moins un paramètre du vaisseau sanguin pendant l'insertion de la canule (117) et que le système comprend en outre des moyens de contrôle permettant de contrôler le mouvement de la canule, à l'aide de moyens d'attache (116) en réponse au suivi du paramètre de vaisseau sanguin.

9. Système selon la revendication 6, **caractérisé en ce qu'**il comprend des moyens d'actionnement adaptés pour déplacer de manière autonome les moyens d'attache dans une position d'insertion (126) et pour insérer l'extrémité distale (122) de la canule dans le vaisseau sanguin, à l'endroit de la ponction.

10. Système selon la revendication 1, **caractérisé en ce que** les moyens de détermination de l'emplacement comprennent des moyens de détection opto-acoustiques, optiques et/ou acoustiques et des moyens de saisie de signal (108, 110) et dans lequel les moyens de traitement (110, 112) sont adaptés pour réaliser le traitement du signal.

11. Système selon la revendication 10, **caractérisé en ce que** les moyens de détection et de saisie du signal (108, 110) sont mis en oeuvre en utilisant des techniques de saisie d'image et de traitement d'image et/ou en utilisant des techniques de détermination de l'emplacement mises en application par le biais d'un Doppler.

12. Système selon la revendication 10, **caractérisé en ce que** les moyens de traitement sont en outre adaptés pour déterminer la position d'insertion (126), en réponse à la position de la ponction (124), obtenue par le biais d'une procédure d'optimisation représentant au moins un paramètre de vaisseau sanguin découlant du rendement desdits moyens de détermination de l'emplacement.

13. Système selon la revendication 9, **caractérisé en ce que** l'orientation de la canule (117) et la direction d'insertion (120) sont modifiables et que les moyens d'actionnement sont adaptés pour provoquer la translation et l'alignement des moyens d'attache dans trois directions dans l'espace.

14. Système selon la revendication 1, **caractérisé en ce que** l'insertion de l'extrémité distale de la canule (122) dans le vaisseau sanguin (102) est réalisée de manière autonome, en réponse à la détection permettant d'indiquer que la canule (117) est dans la position d'insertion (126).
